# EUROPEAN PATENT SPECIFICATION

(11) **EP 4 566 522 B1**
(45) Date of publication and mention of the grant of the patent: **01.07.2026**
(21) Application number: 24216806.0
(22) Date of filing: 02.12.2024
(51) Int. Cl.: A61B 5/024, A61B 5/026, G01N 21/49, G02B 27/48, A61B 5/021, A61B 5/0285, A61B 5/08, A61B 5/1455

(54) **DEVICE AND METHOD FOR ANALYZING A SPECKLE PATTERN FROM BIOLOGICAL TISSUE**
VORRICHTUNG UND VERFAHREN ZUR ANALYSE EINES SPECKLEMUSTERS AUS BIOLOGISCHEM GEWEBE
DISPOSITIF ET PROCÉDÉ D'ANALYSE D'UN MOTIF DE CHATOIEMENT À PARTIR D'UN TISSU BIOLOGIQUE

(30) Priority: 08.12.2023 EP 23215211
(43) Date of publication of application: 11.06.2025
(73) Proprietor: Stichting IMEC Nederland, 5656 AE Eindhoven (NL)
(72) Inventor: HERMELING, Evelien, 6027 NT Soerendonk (NL); KLING, Jesse, 5628 ZR Eindhoven (NL); SCHNITZLER, Lena, 5552 LR Valkenswaard (NL)
(74) Representative: AWA Sweden AB

(56) References cited:
- EP-A1- 0 285 314
- US-A1- 2017 188 853
- FERCHER A F ET AL: "Flow visualization by means of single-exposure speckle photography", OPTICS COMMUNICATIONS, ELSEVIER, AMSTERDAM, NL, vol. 37, no. 5, 1 June 1981 (1981-06-01), pages 326 - 330, XP022599581, ISSN: 0030-4018, [retrieved on 19810601], DOI: 10.1016/0030-4018(81)90428-4
- HERRANZ OLAZÁBAL JORGE ET AL: "Camera-Derived Photoplethysmography (rPPG) and Speckle Plethysmography (rSPG): Comparing Reflective and Transmissive Mode at Various Integration Times Using LEDs and Lasers", vol. 22, no. 16, 13 August 2022 (2022-08-13), CH, pages 6059, XP093068868, ISSN: 1424-8220, Retrieved from the Internet <URL:https://www.mdpi.com/journal/sensors/> DOI: 10.3390/s22166059

## Description

### Technical field

The present disclosure relates to optical analysis of biological tissue, and more specifically to a device and a method for analyzing a speckle pattern from biological tissue.

### Background

Speckles are interference patterns generated when a coherent light source, such as a laser, is used to illuminate a surface. Although often regarded as noise, speckles can also provide useful information. When the illuminated surface is static the speckle pattern will be static and form bright and dark dots. However, if the surface presents changes in time, such as movements or scattering changes, the speckle pattern will be perturbated.

In for example medical applications, Laser Speckle Contrast Imaging (LSCI) may be used to exploit the blurriness of speckles to extract information from biological tissue, such as tissue perfusion. Images of the speckle pattern may be acquired by an imaging detector, after which the speckles are typically processed to estimate the speckle contrast, which is a measure of the blurriness present in the images. Information on perfusion may be derived from the speckle contrast.

Another approach is Speckle Plethysmography (SPG). SPG allows for analysis of spatiotemporal changes of the speckle pattern. In SPG a time sequence of images may be acquired, and the speckle contrast may be determined for each respective image. A time series similar to Photo Plethysmography (PPG) can be obtained, by analyzing how the speckle contrast in the images changes in time. The method used to extract SPG from videos may be the same used for LSCI, i.e. the speckle contrast.

However, there is a need in the art for further improvements related to optical analysis of biological tissue and thus to analysis of speckle patterns therefrom.

EP 0285314 A1 discloses an ophthalmological diagnosis apparatus comprising a double diffraction optical system provided with lenses and an optical spatial frequency filter, whereby an eye fundus image formed at a first image plane, conjugate with the eye fundus is again formed at a second image plane, a magnifying optical system for expanding the eye fundus image formed on the second image plane. The apparatus further comprises a detecting aperture for detecting movement of laser speckles formed at the image plane of magnifying optical system as fluctuations in the intensity of the speckle light.

US 2017/0188853 A1 discloses an imaging apparatus. The imaging apparatus includes a light source that emits coherent light to image an object. The imaging apparatus also includes an optical apparatus that optically processes light from the object. The optical apparatus has a numerical aperture. The imaging apparatus also includes an imager that receives the light optically processed by the optical apparatus. The imaging apparatus also includes circuitry configured to obtain speckle data based on the light received by the imager and control the numerical aperture of the optical apparatus based on the speckle data.

FERCHER, A.F. et al., "FLOW VISUALIZATION BY MEANS OF SINGLE-EXPOSURE SPECKLE PHOTOGRAPHY", OPTICS COMMUNICATIONS, Elsevier, Amsterdam, NL, Volume 37, no. 5, 1 June 1981, discloses flow visualization by means of single-exposure speckle photography. A photograph of a flow field is taken under laser illumination. If the exposure is short enough, the velocity distribution in the field may be mapped on the photograph as variations in speckle contrast. These contrast variations may be converted to intensity variations by means of a spatial filtering technique, to give a direct picture of the velocity distribution in the flow field. A potential application of this technique to the mapping of retinal blood flow is described.

### Summary of the invention

According to the invention, there is provided a device for analyzing a speckle pattern from biological tissue according to claim 1 and a method for analyzing a speckle pattern from biological tissue according to claim 15, preferred embodiments being specified in the dependent claims 2-14.

### Summary of the disclosure

An objective of the present disclosure is to mitigate, alleviate or eliminate one or more deficiencies in the art and disadvantages singly or in any combination. These and other objectives are at least partly met by the invention as defined in the independent claims. Preferred embodiments are set out in the dependent claims.

According to a first aspect there is provided a device for analyzing a speckle pattern from biological tissue, the device comprising:
a light source configured to generate light for illuminating the biological tissue, such that the light is scattered by the biological tissue and forms the speckle pattern;
a luminescent element comprising a luminescent material, configured for converting the light scattered by the biological tissue into luminescent light, by emission of the luminescent light upon interaction with the light scattered by the biological tissue;
a Fourier transform element configured to transform the luminescent light into a spatial Fourier domain at a Fourier plane of the Fourier transform element;
a spatial bandpass filter arranged at the Fourier plane, the spatial bandpass filter being configured to selectively transmit light at a spatial position of the Fourier plane, the spatial position corresponding to a band of speckle sizes of the speckle pattern;
a photosensitive detector configured to detect the light transmitted through the bandpass filter, wherein a signal output from the photosensitive detector comprises information on contrast of the speckle pattern.

By the term "speckles" is here meant an interference pattern formed when coherent light illuminates a surface. On a static surface, the speckle pattern will be static and form bright and dark dots due to constructive and destructive interference of light. Speckle patterns may be formed when illuminating light from a light source is reflected by the surface, which may be referred to as reflection mode. Further, speckle patterns may be formed when illuminating light from the light source is transmitted through the surface, which may be referred to as transmission mode. In the present context, light illuminating a surface of the biological tissue may exit at an opposite side of the biological tissue, where a speckle pattern may be formed.

Living biological tissue presents movements of the tissue, caused by for example blood flow through the tissue and contraction and dilatation of blood vessels in the tissue. When living biological tissue is illuminated by coherent light, the speckle pattern is perturbed by this movement, such that the speckle pattern is blurred, which may result in reduced speckle contrast of the speckle pattern.

It serves to mention that the term plethysmography refers to measurements of changes in volume. By way of example, in conventional Photo Plethysmography (PPG), not involving speckles, contraction and dilatation of blood vessels as the blood flows through the blood vessels is measured. PPG thus performs a measurement of volume change. However, in conventional Speckle Plethysmography (SPG), wherein speckle contrast is measured, the measurement may be affected also by the movements caused by the blood flow. Therefore, although the term SPG implies measurements of volume change, it should be understood that an SPG signal does not necessarily result solely from volume change but may additionally or alternatively result from other changes of properties such as movements of the tissue and/or changes of scattering properties.

Speckle pattern changes from biological tissue may provide useful information in several applications, such as monitoring of vital signs of a person or an animal. By way of example, this could be used in medical applications. However, it should be understood that monitoring of vital signs may also be used in non-medical applications, such as in applications related to sports activities, exercise, and lifestyle. Given as non-limiting examples, monitoring speckle pattern changes may provide information on heart rate (HR), heart rate variability (HRV), pulse oximetry, blood pressure, vascular distensibility/compliance, pulse arrival time, pulse transit time, pulse wave velocity, blood flow velocity, capillary function, capillary refill time, capillary pressure, respiration rate, perfusion, and blood pressure.

The first aspect relates to a device for analyzing a speckle pattern from biological tissue. The speckle pattern is formed by illumination of the biological tissue by the light from the light source.

By the term "light source" is here meant any unit, device and/or element at which light is generated. By way of example, the light source may be, but is not limited to a laser, a laser diode, a light emitting diode, an incandescent light source, a fluorescent light source, or a combination thereof. In this context the term "light" should be allowed a broad interpretation, not limited to visible electromagnetic radiation. Rather, the term "light" may also include for example ultra-violet light and infra-red light.

By the term "Fourier transform element" is here meant any optical element or unit that may transform collected light into a spatial frequency domain at a Fourier plane. The Fourier transform element transforms the light into the spatial frequency domain, thereby distributing the light based on spatial frequencies such that spatial positions in the Fourier plane corresponds to spatial sizes, or spatial frequencies, of the collected light from the biological tissue. More specifically, the Fourier transform element may distribute the light based on spatial frequencies with the zero frequency in the center of the Fourier plane and higher spatial frequencies towards the outer regions of the Fourier plane. Put differently, the light from the biological tissue may be distributed at the Fourier plane to not form an image of the speckle pattern and the biological tissue. Rather, the light may be distributed at the Fourier plane based on spatial frequencies as in a Fourier transform of such an image.

Given as non-limiting examples, the Fourier transform element may be in the form of a lens, a mirror, a waveguide, a photonic integrated circuit (PIC), a refractive system, a meta material, shaped fluids such as droplets or any other element suitable for transforming light into the spatial frequency domain.

The Fourier transform element may collect light coming directly from the biological tissue. However, it is also conceivable that the Fourier element receives light via another optical component arranged to direct the light from the biological tissue towards the Fourier transform element.

The light collected by the Fourier transform element may be, but is not limited to being, collimated when incident onto the Fourier transform element. For at least some Fourier transform elements, collimated light may provide improved performance of the Fourier transform element. By way of example, collimated light may be provided by a collimating optical element being arranged in the light path from the biological tissue to the Fourier transform element. By way of further example, collimated light may be provided by a distance between the biological tissue and the Fourier transform element being sufficiently large for the light reaching the Fourier transform element to be substantially collimated.

By the term "spatial bandpass filter" is here meant any element, unit or device configured to block light from being transmitted through the filter at some spatial positions of the spatial bandpass filter and to allow transmission of light through at least one spatial position of the spatial bandpass filter. Since light at different spatial positions of the Fourier plane represent different spatial frequencies of the collected light, blocking some spatial positions of the Fourier plane by means of the spatial bandpass filter will effectively result in frequency filtering.

By the term "band of speckle sizes" is here meant a size range and that the speckles of the speckle pattern have sizes within said size range. One factor that may determine the size of the speckles is the wavelength of the light. It should be realized that speckles typically have sizes much smaller than the biological tissue that is being illuminated. Therefore, the speckles give rise to higher spatial frequencies in the frequency domain than other, larger features of the biological tissue. Thus, in the Fourier plane light corresponding to the band of speckle sizes of the speckle pattern may be incident farther from the center of the Fourier plane, thus in the more peripheral parts of the Fourier plane, than light corresponding to sizes of other, larger features of the biological tissue.

Hence, by using the spatial bandpass filter to block some regions of the spatial Fourier transformed light at the Fourier plane, e.g. the more central parts, and to allow transmission of light at the more peripheral parts of the Fourier plane, information related to the speckles of the speckle pattern is conserved and information related to larger features of the biological tissue is reduced or eliminated. By the present arrangement, the spatial bandpass filter may selectively transmit light at a spatial position of the Fourier plane, the spatial position corresponding to the band of speckle sizes of the speckle pattern. In the manner described above, the collected light from the biological tissue is filtered such that only light representing speckle sizes within the band of the bandpass filter is transmitted through the bandpass filter.

By the term "photosensitive detector" is here meant any detector onto which at least one light sensitive element configured to react to the light intensity impinging onto the light sensitive element, by generating a signal as a response to the light intensity. Typically, the signal is an electrical signal. However, it is conceivable that the signal may be another type of signal than an electrical signal. Given as non-limiting examples, photosensitive detector may be a photodiode or a photo-multiplier tube (PMT). Given as further non-limiting examples, the photosensitive detector maybe a pixel on an image detector such as a charge-coupled device (CCD) or a complementary metal oxide semiconductor (CMOS).

The device may be configured such that the light being transmitted through the spatial bandpass filter may be directly incident onto the light sensitive element or alternatively, the light may be focused by a lens or redirected by a mirror such that the light is incident onto the light sensitive element of the photosensitive detector. By the present arrangement, light corresponding to the spatial sizes or frequencies of the speckle pattern may reach the photosensitive detector, whereas light corresponding to larger features of the biological tissue is reduced or eliminated. The signal output from the photosensitive detector comprises information on contrast of the speckle pattern. More specifically, the signal output from the photosensitive detector may be proportional to the speckle contrast as calculated according to conventional SPG.

It should be understood that the device may be configured such that the detector and the light source are arranged substantially on the same side of the biological tissue. In such an arrangement the detector may detect the speckle pattern by the light being reflected by the biological tissue. This is referred to as reflection mode. However, it is equally conceivable that the device may be configured such that the detector and the light source are arranged substantially on opposite sides of the biological tissue. In such an arrangement, the detector may detect the speckle pattern of the light having propagated through the biological tissue. This is referred to as transmission mode.

It serves to mention that a conventional approach to SPG typically uses an image detector to acquire time sequences of images of the speckle pattern. The approach of using an image detector may be both bulky as well as expensive. Conventional Laser Speckle Contrast Imaging (LSCI) may be calculated from each pixel within an image sequence by calculation of speckle contrast over time. For this a high framerate is needed, as for example higher than 200 Hz. This alternative approach may also be expensive due to the high framerate required, and may further be challenging to apply, especially when more than one light wavelength is used, due to multiplexing. Both of these conventional approaches to SPG require calculations of the speckle contrast to be made from the acquired data, which may be computationally heavy. Further, both approaches may be sensitive to noise since they are not selective to the particular sizes of the speckles. Light intensity variations from other features may thus cause interference or errors in calculations of speckle contrast.

An advantage is that the device according to the first aspect does not require an image detector to determine information on contrast of the speckle pattern. Nor does the device require high sampling frequencies. By using a single photosensitive detector, a compact device for analyzing a speckle pattern from biological tissue may be provided at a low cost.

Another advantage is that the device directly determines the contrast of the speckles of the speckle pattern. The signal output from the photosensitive detector comprises this information, since the Fourier transform element and the spatial bandpass filter have already processed the optical signal. Consequently, in this manner, no heavy and time-consuming data processing is required.

Yet another advantage is that the spatial bandpass filter allows for selective filtering of speckles in a defined size range which thereby reduces the amount of noise reaching the photosensitive detector. Thus, a device for analyzing a speckle pattern from biological tissue less sensitive to noise may be provided.

According to an embodiment, the Fourier transform element comprises a lens.

By way of example, the lens may be a positive lens. The light scattered by the biological tissue incident onto a surface of the lens may be collected by the lens focusing the light to a focal plane of the lens. In the present arrangement, the focal plane may be the Fourier plane of the lens.

An advantage with this embodiment is that the Fourier transform element may be easily implemented as a lens, yet a lens may efficiently function as a Fourier transform element.

According to an embodiment, the lens is a positive, spherical, plano-convex lens.

It should be understood that in case the Fourier transform element is a spherical lens, the light may be distributed based on spatial frequencies with the zero frequency in the center of the Fourier plane, and with gradually higher spatial frequencies at a gradually increasing radial distance from the center of the Fourier plane. In other words, the light along a circle at a radial distance from the center of the Fourier plane, may correspond to the same spatial frequency, or a narrow band of spatial frequencies. Thus, by a spherical lens, the distribution of spatial frequencies may be circular symmetric.

An advantage with this embodiment is that a spherical lens may provide a simple and intuitive distribution of spatial frequencies. This in turn may facilitate filtering of spatial frequencies.

According to an embodiment, the Fourier transform element comprises any one of a waveguide, a photonic integrated circuit, PIC, a spatial light modulator, or a micro-electro-mechanical system (MEMS) mirror.

By the term "waveguide" is here meant any unit, device and/or element within which light may be guided, and within which transmission is restricted to a single direction, thereby providing transmission with low loss. By way of example, light may be reflected on inner walls of the waveguide by total internal reflection or by means of a reflective coating provided on the walls of the waveguide. Given as non-limiting examples, the waveguide may be a transparent dielectric waveguide or an optical fiber.

By way of example, the Fourier transform element may comprise waveguides configured to create phase shifts for the spatial Fourier transform. Different phase shifts at different waveguides may be providing different delays of the light by different optical path lengths through the waveguides. By way of example, different optical path lengths may be provided by different waveguide lengths or waveguides with different refractive indices. Alternatively, different phase shifts may be provided by the use of one or more lenses.

A PIC may comprise one or more waveguides to provide the phase shifts.

The device for analyzing a speckle pattern from biological tissue further comprises a luminescent element comprising a luminescent material. The luminescent element is arranged such that the light scattered by the biological tissue reaches the luminescent element prior to the light being transformed into the spatial frequency domain by the Fourier transform element.

By way of example, the luminescent material may be a phosphorescent material. By way of further example, the luminescent element may be provided in the form of a sheet of material.

The light scattered by the biological tissue may interact with the luminescent material, whereby emission of luminescent light from the luminescent material is induced. By the present arrangement, the light scattered by the biological tissue is converted into the luminescent light. Thus, the converted scattered light, i.e. the luminescent light, may subsequently be transformed into the spatial frequency domain by the Fourier transform element.

It is conceivable that the luminescent element may be a separate element in the device. In such an arrangement, the luminescent element may be arranged in the light path from the biological tissue to the Fourier transform element, in between the biological tissue and the Fourier transform element. Thus, the light scattered by the biological tissue may first be converted to luminescent light, after which the luminescent light reaches the Fourier transform element. However, it is equally conceivable that the luminescent element may form part of the Fourier transform element. In such an arrangement, the unconverted the light scattered by the biological tissue may reach the Fourier transform element, and may subsequently be converted into luminescent light by the luminescent element of the Fourier transform element, prior to being transformed into the spatial frequency domain by the Fourier transform element.

The luminescent material may be configured to provide a delay of the collected light or to prolong the emission of the collected light. In the manner described above, the luminescent element may emulate an exposure time which may result in a blurring of the speckle pattern, similar to the blurring of the speckle pattern normally seen in conventional SPG measurements caused by the exposure time of the camera used.

According to an embodiment, the spatial bandpass filter is a static bandpass filter configured to selectively transmit light at a predefined spatial position of the Fourier plane.

By the term "static bandpass filter" is here meant a bandpass filter having fixed positions for transmitting and blocking of light. Given as a non-limiting example, a static bandpass filter may be in the form of a mask comprising an opaque portion that prevents light incident onto one side of the filter from being transmitted through the bandpass filter, and further comprising an at least partially transparent portion that allows at least part of the light to be transmitted through the bandpass filter. By way of example, the bandpass filter may be, but is not limited to, a plate with one or more through holes, a transparent glass plate partially painted or otherwise provided with a non-transparent surface coating, or an arrangement of one or more optical components refracting and/or reflecting the light so as to only allow transmission at certain positions.

It should be understood that by a portion being "opaque" is here meant that the portion is opaque with respect to the light generated by light source. By way of example, the light source may generate light at one or more specific wavelengths, and thus by a portion being "opaque" is here meant that the portion is opaque to at least these one or more wavelengths. A portion that is opaque to at least these one or more wavelengths may or may not be transparent or partially transparent to some other wavelengths, not generated by the light source.

Analogous, it should be understood that the term "transparent" is here meant transparent with respect to the light generated by the light source.

An advantage with this embodiment is that a static bandpass filter is easy to implement, and may be implemented at a low cost.

According to an embodiment, the spatial bandpass filter is an annular obstruction aperture.

By the term "annular obstruction aperture" is here meant an obstruction mask that is circularly symmetric. By way of example, an annular obstruction aperture may be a mask configured to allow transmission of light through the filter at an interval between a first radial distance and a second radial distance from the center of the filter, the second radial distance being larger than the first radial distance, and to block light from being transmitted through the filter outside said interval. The spatial bandpass filter may thus present a ring shaped transmission area.

An advantage with this embodiment is that, in case the distribution of spatial frequencies is circular symmetric, an annular obstruction aperture may be an easily implemented yet efficient manner to implement a spatial bandpass filter to allow transmission of only a spatial frequency band corresponding to the band of speckle sizes of the speckle pattern.

According to an embodiment, the spatial bandpass filter is a dynamic bandpass filter configured to selectively transmit light at a variable spatial position of the Fourier plane.

By the term "dynamic bandpass filter" is here meant a bandpass filter having a variable spatial positions for transmitting and blocking of light. By way of example, dynamically variation of the spatial position may be achieved by mechanical, electrical, or electro-optical means, or a combination thereof.

Given as a non-limiting example, a dynamic bandpass filter may be provided by a bandpass filter configured to change position in the Fourier plane. By said arrangement, by moving the bandpass filter to different positions, different spatial bands in Fourier plane and hence different speckle sizes may be selected for transmission. Such a dynamic bandpass filter may be a movable obstruction aperture.

Given as another non-limiting example, a dynamic bandpass filter may comprise a plurality of openable and closable elements distributed across the dynamic bandpass filter, such that by opening an element light will be allowed to be transmitted at the position of the element. Thus, by selectively opening certain element, selection of frequency band and thus band of speckle sizes may be made. By the present arrangement, a signal corresponding to SPG may be provided.

It serves to mention that if all elements were to be open simultaneously, no spatial frequency band would be blocked and all spatial frequencies may be transmitted. In this manner the signal output of the photosensitive element may correspond to that of PPG.

An advantage with this embodiment is that a flexible spatial bandpass filter may be provided. The dynamic bandpass filter may be adjustable to different monitoring situations, such as different distances, different wavelengths, type of biological tissue, size of monitored area of the biological tissue. The dynamic bandpass filter may be adjustable to monitoring different properties of the biological tissue.

According to an embodiment, the spatial bandpass filter comprises one of a dynamically translucent screen, a spatial light modulator, or a micro-electro-mechanical system (MEMS) mirror.

Given as a non-limiting example, the spatial bandpass filter may comprise an electronic device with a plurality of elements wherein each of the elements may be individually programmable in terms of level of transparency. Thereby, portions with different levels of transparency may be dynamically provided to generate the spatial bandpass filter. By the present arrangement, a spatial bandpass filter wherein the spatial position at which light may be transmitted can be altered via electrical signals and thereby be adapted to different spatial frequencies and thus to different speckle sizes.

By way of example, a dynamic translucent screen may be a liquid crystal panel. The liquid crystal panel may comprise a plurality of elements, or pixels, distributed across the surface of the panel.

Given as another non-limiting example, the spatial bandpass filter may comprise a MEMS mirror. The different elements of the MEMS mirror may be directed so as to reflect light such that the reflected light is either incident onto the photosensitive detector or not incident onto the photosensitive detector. Thus, depending on at what position the light reaches the MEMS mirror, light may selected to either reach the photosensitive detector or prevented from reaching the photosensitive detector.

An advantage is that the spatial bandpass filter may allow simple electronic selection of the band to be transmitted, by opening only the elements at the position of the Fourier plane corresponding to the spatial frequency band to be transmitted.

Another advantage is that the spatial bandpass filter may provide an easily implementable dynamic bandpass filter. The transmission band of the filter may be easily selected and changed, by opening and closing respective elements of the liquid crystal panel, thereby allowing transmission of light at a variable spatial position of the Fourier plane.

According to an embodiment, the spatial bandpass filter is a first bandpass filter, and wherein the device further comprises at least a second bandpass filter.

The device may comprise a plurality of bandpass filters, of which the first and the second bandpass form part.

By way of example, a device comprising a plurality of spatial bandpass filters may allow acquisition of spatial band information from multiple targets of biological tissue at a different spatial locations to be made. The spatial band of each spatial bandpass filter may be configured for the respective location of the respective target of biological tissue.

By way of further example, a device comprising a plurality of spatial bandpass filters may allow the use of a plurality of different wavelengths.

By the present arrangement, tissue characterization may be enabled, as for example distinguishing between tissue with oxygenated and deoxygenated hemoglobin. Moreover, the present arrangement may be use for understanding and/or correcting for motion artifacts. Further, the present arrangement may be use for observations of different tissues at different depths, such as capillaries and arteries.

According to an embodiment, the device is configured for remote sensing and is configured to illuminate the biological tissue and to collect the light scattered by the biological tissue from a distance to the biological tissue in the interval of 0.1 m to 2 m.

The biological tissue may be a body of a person or an animal. The device may be used to remotely acquire information on contrast of the speckle pattern from the body to monitor for example vital signs at a distance from the body. By the present arrangement, no physical contact between the device and the body is required.

By way of example, the device for remote sensing may be a device wherein the light source, the Fourier transform element, the spatial bandpass filter, and the photosensitive detector are arranged in a common housing. One or more of said parts may be integrated to form a compact device for remote sensing.

By way of further example, the device for remote sensing may alternatively be a device wherein one or more of the light source, the Fourier transform element, the spatial bandpass filter, and the photosensitive detector are remotely arranged to the other parts.

An advantage with a device for remote sensing is that monitoring of contrast of the speckle pattern, and thus of conditions such as vital signs, may be provided without the requirement of physical contact between the device and the body. This in turn may allow for monitoring to be made for a long time without causing any discomfort to the subject.

According to an embodiment, the light source, the Fourier transform element, the spatial bandpass filter, and the photosensitive detector are arranged in a common housing, and wherein the housing is configured to be wearable on a body of a person or an animal.

By way of example, the housing may comprise a textile/garment configured to be worn on the body, or the housing may comprise a belt or strap, configured to be worn around a body part of the body. By way of further example, the housing may be implemented in the form of a clamp configured to be clamped around a body part of the body.

An advantage with this embodiment is that it may provide a convenient manner of wearing the device, such that the device may be worn for a long time and thus providing monitoring for a long time, while minimizing discomfort for the wearer.

According to an embodiment, the signal output from the photosensitive detector further comprises information on an amount of speckles of the speckle pattern.

It serves to mention that the speckle contrast may be considered to be a quantification of blurriness of the speckles of the speckle pattern, for a given number of speckles. The amount of speckles, on the other hand, is a value obtained by counting the number of speckles of the speckle pattern. The amount of speckles as well as the speckle contrast may modulate over time. It may therefore be of interest to monitor also the amount of speckles of the speckle pattern, in addition to monitoring the speckle contrast.

According to an embodiment, the signal output from the photosensitive detector further comprises information on variations of the contrast of the speckle pattern over time.

The variations of the contrast of the speckle pattern over time corresponds to an SPG signal. Thus, the signal output from the photosensitive detector may provide information on movements of living biological tissue caused by for example blood flow through the tissue and contraction and dilatation of blood vessels in the tissue.

An advantage is that an SPG signal may be provided without an image detector and/or a high sampling frequency. By using a single photosensitive detector, a compact device enabling acquisition of an SPG signal from biological tissue may be provided at a low cost.

Another advantage is that the device directly determines the contrast of the speckles of the speckle pattern. The signal output from the photosensitive detector comprises the information of the SPG signal, since the Fourier transform element and the spatial bandpass filter has already processed the optical signal. Consequently, in this manner, no heavy and time consuming data processing is required to provide the SPG signal.

According to an embodiment, the light source is configured to generate light being at least partially coherent.

Coherent light may be advantageous as it improves the interference and thus the visibility and contrast of the speckles. A coherent light source may be a laser. However, it should be understood that also partially coherent light may provide a speckle pattern with sufficient visibility and contrast. A partially coherent light source may e.g. be a light emitting diode, LED, emitting light through a pinhole onto the biological tissue. A coherent light source may provide better speckle contrast but may be more expensive while a partially coherent light source may provide less speckle contrast but may be less expensive.

According to an embodiment, the light source is configured to generate light of at least two wavelengths.

Given as non-limiting examples, the light source configured to generate at least two wavelengths may be, a laser, such as a white laser, or a supercontinuum laser. Given as another non-limiting example, the light source configured to generate at least two wavelengths may be, a superluminescent diode.

It should be understood that different wavelengths may have different penetration depths within the targeted biological tissue. As previously mentioned, by using at least two wavelengths, physiological information such as blood oxygenation may be acquired. Moreover, the present arrangement may be use for understanding and/or correcting for motion artifacts. Further, the present arrangement may be use for observations of different tissues at different depths, such as capillaries and arteries.

According to a second aspect there is provided a method for analyzing a speckle pattern from biological tissue, the method comprising:
illuminating the biological tissue with light from a light source, such that the light is scattered by the biological tissue and forms the speckle pattern;
converting, by a luminescent material of a luminescent element, the light scattered by the biological tissue into luminescent light, by emission of the luminescent light upon interaction with the light scattered by the biological tissue;
transforming, by the Fourier transform element, the luminescent light into a spatial Fourier domain at a Fourier plane of the Fourier transform element;
selectively transmit, by a spatial bandpass filter arranged at the Fourier plane, light at a spatial position of the Fourier plane, the spatial position corresponding to a band of speckle sizes of the speckle pattern;
detecting, by a photosensitive detector, the light transmitted though the bandpass filter, wherein a signal output from the photosensitive detector comprises information on contrast of the speckle pattern.

Effects and features of the second aspect are largely analogous to those described above in connection with the first aspect. Embodiments mentioned in relation to the first aspect are largely compatible with the second aspect. It is further noted that the disclosure relates to all possible combinations of features unless explicitly stated otherwise.

Other objectives, features and advantages of the present disclosure will appear from the following detailed description, from the attached claims as well as from the drawings.

### Brief descriptions of the drawings

The above, as well as additional objects, features and advantages of the present disclosure, will be better understood through the following illustrative and non-limiting detailed description, with reference to the appended drawings. In the drawings like reference numerals will be used for like elements unless stated otherwise.
Fig. 1A schematically illustrates a device for analyzing a speckle pattern from biological tissue in reflection mode.
Fig. 1B illustrates the signal output from the photosensitive detector of the device.
Fig. 1C schematically illustrates a device for analyzing a speckle pattern from biological tissue in transmission mode.
Fig. 2A schematically illustrates a device 200 for remotely analyzing a speckle pattern from biological tissue.
Fig. 2B schematically illustrated a device to be clamped around the finger, for analyzing a speckle pattern.
Fig. 3 illustrates a schematic block diagram shortly summarizing the method for analyzing a speckle pattern from biological tissue.

### Detailed description

In cooperation with attached drawings, the technical contents and detailed description of the present concept are described thereinafter according to a preferable embodiment, being not used to limit the claimed scope. This concept may be embodied in many different forms and should not be construed as limited to the embodiments set forth herein; rather, these embodiments are provided for thoroughness and completeness, and fully convey the scope of the concept to the skilled person.

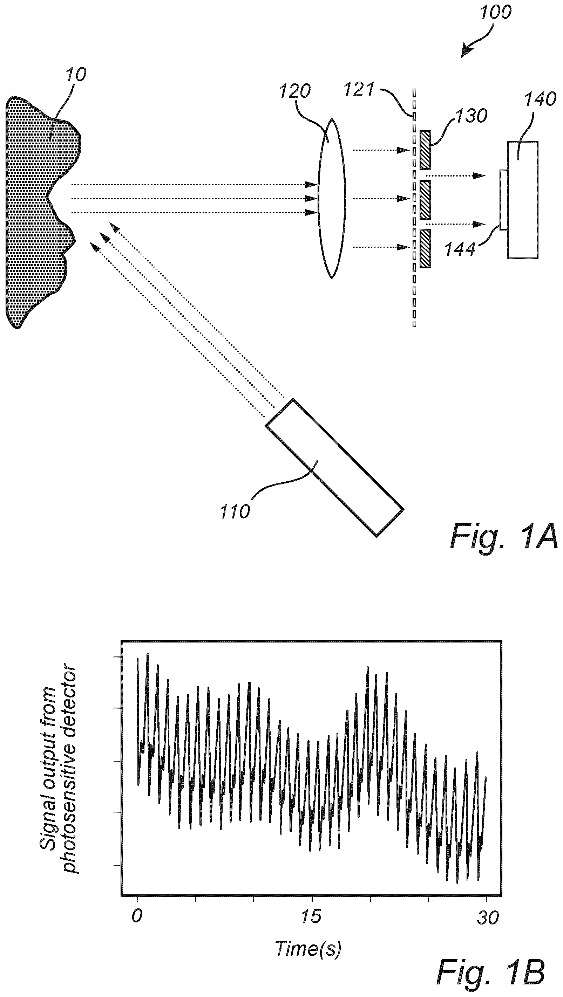

Fig. 1A schematically illustrates a device 100 for analyzing a speckle pattern from biological tissue 10. The device 100 operates in reflection mode.

The device 100 comprises a light source 110 configured to generate light for illuminating the biological tissue 10. In the present example, the light source 110 is a diode laser configured to generate at least partially coherent light. However, it is conceivable that the light source 110 may be of another type. Further, in the present example, the light source 110 is configured to generate light with a single wavelength. However, it is conceivable that the light source 110 alternatively may be configured to generate light of at least two wavelengths such as a plurality of wavelengths. The light from the light source 110 illuminating the biological tissue 10 is scattered by the biological tissue 10 and forms a speckle pattern.

By monitoring the speckle pattern, movements of living biological tissue caused by for example blood flow through the tissue and contraction and dilatation of blood vessels in the tissue may be monitored. The speckle pattern is perturbed by the movements, resulting in varied speckle sizes and location of the speckle pattern. However, the speckle pattern is superimposed on the biological tissue also featuring light intensity variations. Light intensity variations from other features may cause interference or errors in further analysis of the speckle pattern, and it is therefore desirable to reduce or even eliminate such intensity variations, prior to further analysis.

The device 100 further comprises a Fourier transform element 120. In the present example, the Fourier transform element 120 is a positive, spherical lens. However, it should be understood that the Fourier transform element 120 may be of another type, such that the Fourier transform element 120 may comprise a waveguide or a photonic integrated circuit, PIC.

The Fourier transform element 120 is configured to collect the light scattered by the biological tissue 10. The collected light is subsequently transformed into the spatial frequency domain at a Fourier plane 121 of the Fourier transform element 120.

At the Fourier plane 121, the light from the biological tissue 10 is not distributed to form an image of the speckle pattern and the biological tissue 10. Instead, the light is distributed based on spatial frequencies as in a Fourier transform of such an image. In other words, the light will be distributed with the zero frequency in the center of the Fourier plane, and with gradually higher spatial frequencies at a gradually increasing radial distance from the center of the Fourier plane. Thus, by a spherical lens, the distribution of spatial frequencies may be circular symmetric.

It should be realized that the device 100 comprises a luminescent element (not illustrated here). The luminescent element converts the light scattered by the biological tissue 10 into luminescent light, prior to the light being transformed into the spatial frequency domain by the Fourier transform element 120. The luminescent element may be arranged in the light path between the biological tissue 10 and the Fourier transform element 120, or the luminescent element may alternatively form part of the Fourier transform element 120.

In order to filter the light based on spatial frequencies, such that only light corresponding to a band of speckle sizes of the speckle pattern is transmitted, the device 100 comprises a spatial bandpass filter 130. In the present example, the spatial bandpass filter 130 is a static bandpass filter in the form of is an annular obstruction aperture. The spatial bandpass filter 130 is configured to selectively transmit light at a predefined radial distance from the center of the spatial bandpass filter 130. The spatial bandpass filter 130 is arranged at the Fourier plane 121. With the center of spatial bandpass filter 130 arranged along an optical axis of the Fourier transform element 120, the center of the spatial bandpass filter 130 may coincide with the center of the Fourier plane 121. Thus, by the present arrangement, the spatial bandpass filter 130 may transmit light at a predefined radial distance from the center of the Fourier plane 121. Due to the circular symmetry of the frequency distribution of the light, the light along a circle at the radial distance from the center of the Fourier plane 121, may correspond to the same spatial frequency, or a narrow band of spatial frequencies. Hence, in the manner described above, the selected band of spatial frequencies may be transmitted through the spatial bandpass filter 130, whereas other spatial frequencies may instead be blocked by the spatial bandpass filter 130. The radial distance may be selected such that the spatial position allowing light to be transmitted, corresponds to the band of speckle sizes of the speckle pattern.

It serves to mention that the spatial bandpass filter 130 does not necessarily need to be a static bandpass filter. By way of example, the spatial bandpass filter 130 may alternatively be a dynamic bandpass filter configured to selectively transmit light at a variable spatial position of the Fourier plane 121, such as a liquid crystal panel, a spatial light modulator, or a micro-electro-mechanical system (MEMS) mirror.

Although not illustrated here, it is further conceivable that the spatial bandpass filter 130 is a first bandpass filter, and wherein the device further comprises at least a second bandpass filter.

The device 100 further comprises a photosensitive detector 140. The photosensitive detector 140 comprises a light sensitive element 144 configured to react to the light intensity impinging onto the light sensitive element 144, by generating an electrical signal as a response to the light intensity. In the present example, the photosensitive detector 140 is a photodiode, however, it is conceivable that also other types of photosensitive detectors may be suitable.

The photosensitive detector 140 is arranged such that the light being transmitted though the bandpass filter 130 impinges onto the light sensitive element 144. By the present arrangement, since spatial frequency filtering is provided by the Fourier transform element 120 and the spatial bandpass filter 130 arranged in the Fourier plane 121, only light corresponding to the spatial sizes or frequencies of the speckle pattern may reach the photosensitive detector 140, whereas light corresponding to larger features of the biological tissue is reduced or eliminated. By the present arrangement, the signal output from the photosensitive detector 140 may comprise information on contrast of the speckle pattern. It should be realized that, in addition to information on contrast of the speckle pattern, the signal output from the photosensitive detector 140 may optionally comprise also information on an amount of speckles of the speckle pattern.

Fig. 1B illustrates the signal output from the photosensitive detector 140 of the device 100 as illustrated in Fig. 1A. Fig. 1B illustrates signal output variations due to variations of the contrast of the speckle pattern over time. Movements of the living biological tissue 10 may be caused by for example blood flow through the tissue 10 and contraction and dilatation of blood vessels in the tissue 10. Reduced speckle contrast may result in less light being transmitted through the spatial bandpass filter 130, which is detected by the photosensitive detector 140 as a temporary reduction of signal. Thus, the signal output of the photosensitive detector 140 corresponds to that of conventional SPG.

In conventional SPG analysis, a time sequence of images of the speckle pattern may be acquired. An average speckle contrast may then be determined for each respective image of the time sequence. Determining an average speckle contrast typically comprises determining a plurality of speckle contrasts in a plurality of local neighborhoods in the image, wherein a local neighborhood may comprise only a few adjacent picture elements as compared to the full image. Typically, the speckle contrast of a local neighborhood is determined by calculating the standard deviation in the local neighborhood of a picture element, and subsequently divide the standard deviation by the average intensity of that neighborhood. The average speckle contrast for the image is determined by averaging of said plurality of speckle contrasts of the local neighborhoods. In the present manner, a single value for the image is calculated. From the average speckle contrasts of each of the images in the time sequence of images, an SPG time domain signal may be determined.

However, with the device of the present disclosure, no imaging of the speckle pattern is required, as in conventional SPG, and thus no computationally demanding image processing is required, since the optical hardware of the device 100 provides substantially instantaneous processing of the light and provides the SPG signal directly as an output from the photosensitive detector 140.

The device 100 as described in relation to Fig. 1A operates in reflection mode. However, it is conceivable that a device for analyzing a speckle pattern from biological tissue may alternatively be configured to operate in transmission mode. It should be understood that the signal output from the photosensitive detector 140 as illustrated in Fig. 1B, is equally representative for a device operating in reflection mode and a device operating in transmission mode.

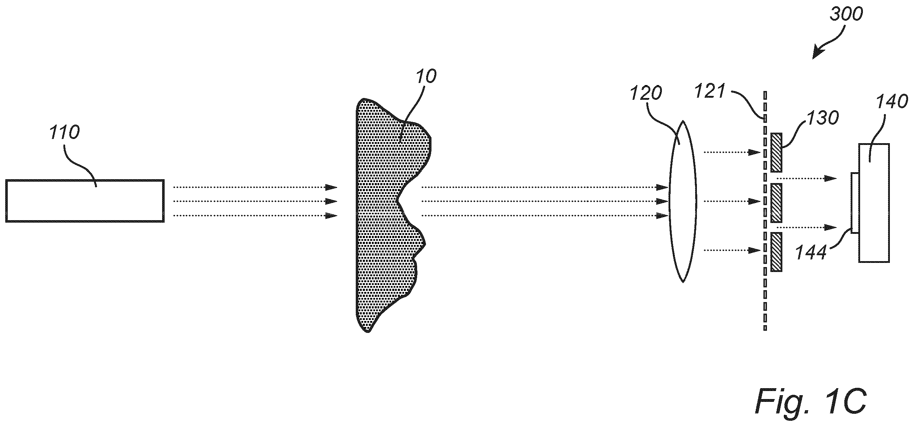

Fig. 1C schematically illustrates a device 300 for analyzing a speckle pattern from biological tissue 10. The device 300 operates in reflection mode.

Similar to the device 100, the device 300 comprises a light source 110 configured to generate light for illuminating the biological tissue 10. The light source 110 is arranged such that the light generated by the light source 110 is directed towards a side of the biological tissue 10. By the present arrangement, the light source 110 illuminates through the biological tissue 10, such that the light forms the speckle pattern on an opposite side of the biological tissue 10.

The device 300 further comprises a Fourier transform element 120. The Fourier transform element 120 is configured to collect the light scattered at the surface of the biological tissue 10 after having propagated through the biological tissue 10. The collected light is subsequently transformed into the spatial frequency domain at a Fourier plane 121 of the Fourier transform element 120.

The device 300 further comprises a spatial bandpass filter 130 arranged at the Fourier plane 121. The spatial bandpass filter 130 is configured to selectively transmit light at a selected band of spatial frequencies through the spatial bandpass filter 130. The band of spatial frequencies may be selected to correspond to the band of speckle sizes of the speckle pattern.

The device 300 further comprises a photosensitive detector 140 with a light sensitive element 144. The photosensitive detector 140 is arranged such that the light being transmitted though the bandpass filter 130 impinges onto the light sensitive element 144. By the present arrangement, since spatial frequency filtering is provided by the Fourier transform element 120 and the spatial bandpass filter 130 arranged in the Fourier plane 121, only light corresponding to the spatial sizes or frequencies of the speckle pattern may reach the photosensitive detector 140, whereas light corresponding to larger features of the biological tissue is reduced or eliminated. By the present arrangement, the signal output from the photosensitive detector 140 may comprise information on contrast of the speckle pattern. It should be realized that, in addition to information on contrast of the speckle pattern, the signal output from the photosensitive detector 140 may optionally comprise also information on an amount of speckles of the speckle pattern.

It serves to mention that the illumination power required to form a speckle pattern in transmission mode is typically higher than required for the corresponding situation in reflection mode. The reason for this requirement is at least partially due to multiple scattering of the light as it travels through the biological tissue, with the effect that smearing out the coherence of the light and thus the otherwise distinct pattern of bright and dark dots. A speckle pattern may be formed if a high enough illumination power is used.

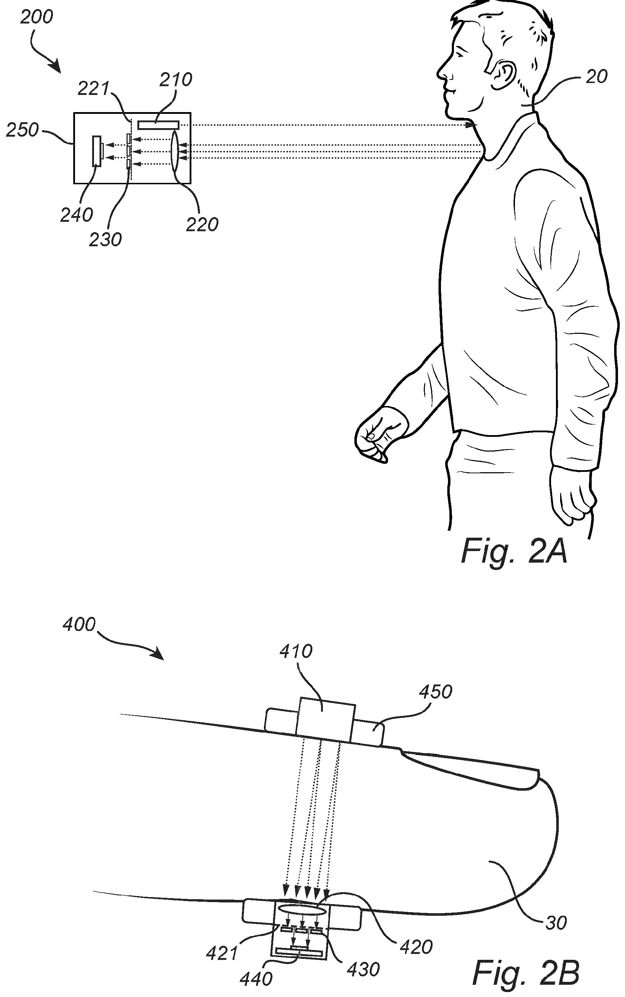

Fig. 2A schematically illustrates a device 200 for remotely analyzing a speckle pattern from biological tissue. In the present example, the biological tissue may be any visible part of the body 20. The device 200 is configured for remote sensing of vital signs of the body 20. By way of example, the device 200 may be configured to be arranged at a distance of 0.1 m to 2 m from the body 20, such as for example 0.5 m from the body 20. Device 200 shares some of the features with device 100 described in relation to Fig. 1A, the details of which are not repeated here.

The device 200 comprises a light source 210 configured to generate light for illuminating the body 20. The device 200 may be arranged such that the light generated by the light source 210 is directed towards the body 20. Light illuminating the body 20 may be scattered by the body 20 forming a speckle pattern on the body 20. At least some of the light forming the speckle pattern may be scattered back towards the device 200.

The device 200 further comprises a Fourier transform element 220 configured to collect the light scattered by the body 20, and to transform the collected light into the spatial frequency domain at a Fourier plane 221 of the Fourier transform element 220.

In order to filter the light based on spatial frequencies, such that only light corresponding to the band of speckle sizes of the speckle pattern is transmitted, the device 200 comprises a spatial bandpass filter 230. With the spatial bandpass filter 230 arranged at the Fourier plane 221, light at a predefined position, e.g. radial distance from the center, of the Fourier plane 221 may be transmitted. The light being transmitted through the spatial bandpass filter 230 may correspond to a narrow band of spatial frequencies, corresponding to the band of speckle sizes of the speckle pattern.

The device 200 further comprises a photosensitive detector 240, in the present example a photodiode, arranged such that the light being transmitted though the bandpass filter 230 impinges onto the photosensitive detector 240. By the present arrangement, the signal output from the photosensitive detector 240 may comprise information on contrast of the speckle pattern.

Hence, in the manner described above, speckle pattern changes from the body 20 may be monitored remotely, which may provide useful information of e.g. vital signs of the person or the animal, without the need for physical contact with the person or the animal.

The device 200 may comprise a common housing 250 in which the light source 210, the Fourier transform element 220, the spatial bandpass filter 230, and the photosensitive detector 240 are arranged. By the present arrangement, a compact and stable device 200 for analyzing a speckle pattern from biological tissue 20 by remote sensing, may be provided.

It serves to mention that a device for analyzing a speckle pattern from biological tissue may not necessarily be a device for remote sensing. As an alternative, a device for analyzing a speckle pattern from biological tissue may be a device wherein the light source, the Fourier transform element, the spatial bandpass filter, and the photosensitive detector are arranged in a common housing, and wherein the housing is configured to be wearable on a body of a person or an animal. It is conceivable that with a housing wearable on the body, the speckle pattern may be analyzed in either reflection mode or in transmission mode.

Fig. 2B schematically illustrates a device 400 for analyzing a speckle pattern from biological tissue. In the present example, the biological tissue may be a finger 30. The device 400 comprises a housing 450 configured to be wearable on the finger 30. The housing 450 is implemented in the form of a clamp or a finger cuff to be clamped around the finger 30. Device 400 shares some of the features with device 300 described in relation to Fig. 1C, the details of which are not repeated here.

The device 400 further comprises a light source 410 configured to generate light for illuminating through the finger 30. The light source 410 is arranged in the housing 450 such that the light generated by the light source 410 is directed towards a side of the finger 30, when the device 400 is attached to the finger 30. By the present arrangement, the light source 410 may illuminate through the finger 30, such that the light forms the speckle pattern on an opposite side of the finger 30.

The device 400 further comprises a Fourier transform element 420 configured to collect the light scattered at the side of the finger 30 opposite to the light source 410, and to transform the collected light into the spatial frequency domain at a Fourier plane 421 of the Fourier transform element 420.

The device 400 further comprises a spatial bandpass filter 430 arranged at the Fourier plane 421. The spatial bandpass filter 430 is configured to selectively transmit light at a selected band of spatial frequencies through the spatial bandpass filter 430. The band of spatial frequencies may be selected to correspond to the band of speckle sizes of the speckle pattern.

The device 400 further comprises a photosensitive detector 440, in the present example a photodiode, arranged such that the light being transmitted though the bandpass filter 430 impinges onto the photosensitive detector 440. By the present arrangement, the signal output from the photosensitive detector 440 may comprise information on contrast of the speckle pattern.

By the present arrangement, speckle pattern changes from the finger 30 may be monitored, which may provide useful information in several applications, such as vital signs of the person. Given as a non-limiting example, the present arrangement may be used in medical applications for monitoring a patient.

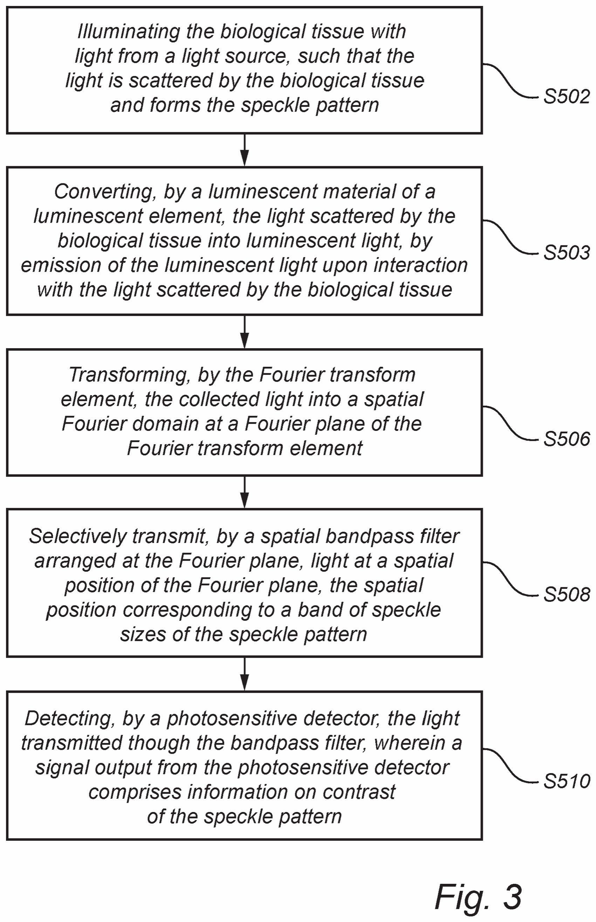

Fig. 3 illustrates a schematic block diagram shortly summarizing the method for analyzing a speckle pattern from biological tissue. It should be understood that the steps of the method, although listed in a specific order herein, may be performed in any order suitable.

The method may comprise illuminating S502 the biological tissue with light from a light source, such that the light is scattered by the biological tissue and forms the speckle pattern.

The method may comprise converting S503, by a luminescent material of a luminescent element, the light scattered by the biological tissue into luminescent light, by emission of the luminescent light upon interaction with the light scattered by the biological tissue.

The method may comprise transforming S506, by the Fourier transform element, the luminescent light into a spatial Fourier domain at a Fourier plane of the Fourier transform element.

The method may comprise selectively transmit S508, by a spatial bandpass filter arranged at the Fourier plane, light at a spatial position of the Fourier plane, the spatial position corresponding to a band of speckle sizes of the speckle pattern.

The method may comprise detecting S510, by a photosensitive detector, the light transmitted though the bandpass filter, wherein a signal output from the photosensitive detector comprises information on contrast of the speckle pattern.

In the above the concept has mainly been described with reference to a limited number of examples. However, as is readily appreciated by a person skilled in the art, other examples than the ones disclosed above are equally possible, the scope of the invention being defined by the appended claims.

## Claims

1. A device (100, 200, 300, 400) for analyzing a speckle pattern from biological tissue (10), the device (100, 200, 300, 400) comprising:
a light source (110, 210, 410) configured to generate light for illuminating the biological tissue (10), such that the light is scattered by the biological tissue (10) and forms the speckle pattern;
a luminescent element comprising a luminescent material, configured for converting the light scattered by the biological tissue (10) into luminescent light, by emission of the luminescent light upon interaction with the light scattered by the biological tissue (10);
a Fourier transform element (120, 220, 420) configured to transform the luminescent light into a spatial Fourier domain at a Fourier plane (121, 221, 421) of the Fourier transform element (120, 220, 420);
a spatial bandpass filter (130, 230, 430) arranged at the Fourier plane (121, 221, 421), the spatial bandpass filter (130, 230, 430) being configured to selectively transmit light at a spatial position of the Fourier plane (121, 221, 421), the spatial position corresponding to a band of speckle sizes of the speckle pattern;
a photosensitive detector (140, 240, 440) configured to detect the light transmitted though the bandpass filter (130, 230, 430), wherein a signal output from the photosensitive detector (140, 240, 440) comprises information on contrast of the speckle pattern.

2. The device (100, 200, 300, 400) according to claim 1, wherein the Fourier transform element (120, 220, 420) comprises a lens.

3. The device (100, 200, 300, 400) according to claim 2, wherein the lens is a positive, spherical, plano-convex lens.

4. The device (100, 200, 300, 400) according to claim 1, wherein the Fourier transform element (120, 220, 420) comprises any one of a waveguide, a photonic integrated circuit, PIC, a spatial light modulator, or a micro-electro-mechanical system (MEMS) mirror.

5. The device (100, 200, 300, 400) according to any of the preceding claims, wherein the spatial bandpass filter (130, 230, 430) is a static bandpass filter configured to selectively transmit light at a predefined spatial position of the Fourier plane (121, 221, 421).

6. The device (100, 200, 300, 400) according to claim 5, wherein the spatial bandpass filter (130, 230, 430) is an annular obstruction aperture.

7. The device (100, 200, 300, 400) according to any one of claims 1 to 4, wherein the spatial bandpass filter (130, 230, 430) is a dynamic bandpass filter configured to selectively transmit light at a variable spatial position of the Fourier plane (121, 221, 421).

8. The device (100, 200, 300, 400) according to claim 7, wherein the spatial bandpass filter (130, 230, 430) comprises one of a dynamically translucent screen, a spatial light modulator, or a micro-electro-mechanical system (MEMS) mirror.

9. The device (100, 200, 300, 400) according to any of the preceding claims, wherein the spatial bandpass filter (130, 230, 430) is a first bandpass filter, and wherein the device (100, 200, 300, 400) further comprises at least a second bandpass filter.

10. The device (200) according to any of the preceding claims, wherein the device (200) is configured for remote sensing and is configured to illuminate the biological tissue and to collect the light scattered by the biological tissue from a distance to the biological tissue in the interval of 0.1 m to 2 m.

11. The device (100, 200, 300, 400) according to any of the preceding claims, wherein the signal output from the photosensitive detector (140, 240, 440) further comprises information on an amount of speckles of the speckle pattern.

12. The device (100, 200, 300, 400) according to any of the preceding claims, wherein the signal output from the photosensitive detector (140, 240, 440) further comprises information on variations of the contrast of the speckle pattern over time.

13. The device (100, 200, 300, 400) according to any one of the preceding claims, wherein the light source (110, 210, 410) is configured to generate light being at least partially coherent.

14. The device (100, 200, 300, 400) according to any one of the preceding claims, wherein the light source (110, 210, 410) is configured to generate light of at least two wavelengths.

15. A method for analyzing a speckle pattern from biological tissue, the method comprising:
illuminating (S502) the biological tissue with light from a light source, such that the light is scattered by the biological tissue and forms the speckle pattern;
converting (S503), by a luminescent material of a luminescent element, the light scattered by the biological tissue into luminescent light, by emission of the luminescent light upon interaction with the light scattered by the biological tissue;
transforming (S506), by a Fourier transform element, the luminescent light into a spatial Fourier domain at a Fourier plane of the Fourier transform element;
selectively transmitting (S508), by a spatial bandpass filter arranged at the Fourier plane, light at a spatial position of the Fourier plane, the spatial position corresponding to a band of speckle sizes of the speckle pattern;
detecting (S510), by a photosensitive detector, the light transmitted though the bandpass filter, wherein a signal output from the photosensitive detector comprises information on contrast of the speckle pattern.

## Patentansprüche

1. Vorrichtung (100, 200, 300, 400) zum Analysieren eines Speckle-Musters aus biologischem Gewebe (10), wobei die Vorrichtung (100, 200, 300, 400) umfasst:
eine Lichtquelle (110, 210, 410), die dazu konfiguriert ist, Licht zum Beleuchten des biologischen Gewebes (10) derart zu generieren, dass das Licht von dem biologischen Gewebe (10) gestreut wird und das Speckle-Muster bildet;
ein lumineszierendes Element, das ein lumineszierendes Material umfasst, das dazu konfiguriert ist, das Licht, das von dem biologischen Gewebe (10) gestreut wird, durch die Emission des lumineszierenden Lichts bei einer Interaktion mit dem Licht, das von dem biologischen Gewebe (10) gestreut wird, in lumineszierendes Licht umzuwandeln,
ein Fourier-Transformationselement (120, 220, 420), das dazu konfiguriert ist, das lumineszierende Licht in eine räumliche Fourier-Domäne auf einer Fourier-Ebene (121, 221, 421) des Fourier-Transformationselements (120, 220, 420) zu transformieren;
ein räumliches Bandpassfilter (130, 230, 430), das auf der Fourier-Ebene (121, 221, 421) eingerichtet ist, wobei das räumliche Bandpassfilter (130, 230, 430) dazu konfiguriert ist, Licht in einer räumlichen Position der Fourier-Ebene (121, 221, 421) selektiv durchzulassen, wobei die räumliche Position einem Band von Speckle-Größen des Speckle-Musters entspricht;
einen lichtempfindlichen Detektor (140, 240, 440), der dazu konfiguriert ist, das Licht zu detektieren, das durch das Bandpassfilter (130, 230, 430) durchgelassen wird, wobei ein Signal, das von dem lichtempfindlichen Detektor (140, 240, 440) ausgegeben wird, Informationen über den Kontrast des Speckle-Musters umfasst.

2. Vorrichtung (100, 200, 300, 400) nach Anspruch 1, wobei das Fourier-Transformationselement (120, 220, 420) eine Linse umfasst.

3. Vorrichtung (100, 200, 300, 400) nach Anspruch 2, wobei die Linse eine positive, sphärische, plankonvexe Linse ist.

4. Vorrichtung (100, 200, 300, 400) nach Anspruch 1, wobei das Fourier-Transformationselement (120, 220, 420) eines umfasst von einem Wellenleiter, einer photonischen integrierten Schaltung, PIC, einem räumlichen Lichtmodulator oder einem mikroelektromechanischen System-(MEMS) Spiegel.

5. Vorrichtung (100, 200, 300, 400) nach einem der vorhergehenden Ansprüche, wobei das räumliche Bandpassfilter (130, 230, 430) ein statisches Bandpassfilter ist, das dazu konfiguriert ist, Licht an einer vordefinierten räumlichen Position der Fourier-Ebene (121, 221, 421) selektiv durchzulassen.

6. Vorrichtung (100, 200, 300, 400) nach Anspruch 5, wobei das räumliche Bandpassfilter (130, 230, 430) eine ringförmige Abschattungsblende ist.

7. Vorrichtung (100, 200, 300, 400) nach einem der Ansprüche 1 bis 4, wobei das räumliche Bandpassfilter (130, 230, 430) ein dynamisches Bandpassfilter ist, das dazu konfiguriert ist, Licht in einer variablen räumlichen Position der Fourier-Ebene (121, 221, 421) selektiv durchzulassen.

8. Vorrichtung (100, 200, 300, 400) nach Anspruch 7, wobei das räumliche Bandpassfilter (130, 230, 430) eines von einer dynamisch durchscheinenden Abschirmung, einem räumlichen Lichtmodulator oder einem mikroelektromechanischen System- (MEMS) Spiegel umfasst.

9. Vorrichtung (100, 200, 300, 400) nach einem der vorhergehenden Ansprüche, wobei das räumliche Bandpassfilter (130, 230, 430) ein erstes Bandpassfilter ist, und wobei die Vorrichtung (100, 200, 300, 400) ferner mindestens ein zweites Bandpassfilter umfasst.

10. Vorrichtung (200) nach einem der vorhergehenden Ansprüche, wobei die Vorrichtung (200) zur Fernabtastung konfiguriert ist und dazu konfiguriert ist, das biologische Gewebe zu beleuchten und das Licht zu sammeln, das von dem biologischem Gewebe aus einem Abstand auf das biologische Gewebe in dem Intervall von 0,1 m bis 2 m gestreut wird.

11. Vorrichtung (100, 200, 300, 400) nach einem der vorhergehenden Ansprüche, wobei das Signal, das von dem lichtempfindlichen Detektor (140, 240, 440) ausgegeben wird, ferner Informationen über eine Speckle-Menge des Speckle-Musterns umfasst.

12. Vorrichtung (100, 200, 300, 400) nach einem der vorhergehenden Ansprüche, wobei das Signal, das von dem lichtempfindlichen Detektor (140, 240, 440) ausgegeben wird, ferner Informationen über Variationen des Kontrasts des Speckle-Musters im Verlauf der Zeit umfasst.

13. Vorrichtung (100, 200, 300, 400) nach einem der vorhergehenden Ansprüche, wobei die Lichtquelle (110, 210, 410) dazu konfiguriert ist, Licht zu generieren, das mindestens teilweise kohärent ist.

14. Vorrichtung (100, 200, 300, 400) nach einem der vorhergehenden Ansprüche, wobei die Lichtquelle (110, 210, 410) dazu konfiguriert ist, Licht von mindestens zwei Wellenlängen zu generieren.

15. Verfahren zum Analysieren eines Speckle-Musters aus biologischem Gewebe, wobei das Verfahren umfasst:
Beleuchten (S502) des biologischen Gewebes mit Licht aus einer Lichtquelle, so dass das Licht von dem biologischen Gewebe gestreut wird und das Speckle-Muster bildet;
Umwandeln (S503), durch ein lumineszierendes Material eines lumineszierenden Elements, des Lichts, das von dem biologischen Gewebe gestreut wird, in lumineszierendes Licht, durch die Emission des lumineszierenden Lichts bei einer Interaktion mit dem Licht, das von dem biologischen Gewebe gestreut wird;
Transformieren (S506), durch ein Fourier-Transformationselement, des lumineszierenden Lichts in eine räumliche Fourier-Domäne auf einer Fourier-Ebene des Fourier-Transformationselements;
selektives Durchlassen (S508), durch ein räumliches Bandpassfilter, das auf der Fourier-Ebene eingerichtet ist, von Licht in einer räumlichen Position der Fourier-Ebene, wobei die räumliche Position einem Band von Speckle-Größen des Speckle-Musters entspricht;
Detektieren (S510), durch einen lichtempfindlichen Detektor, des Lichts, das durch das Bandpassfilter durchgelassen wird, wobei ein Signal, das von dem lichtempfindlichen Detektor ausgegeben wird, Informationen über den Kontrast des Speckle-Musters umfasst.

## Revendications

1. Dispositif (100, 200, 300, 400) d'analyse d'un motif de chatoiement d'un tissu biologique (10), le dispositif (100, 200, 300, 400) comprenant :
une source lumineuse (110, 210, 410) configurée pour générer de la lumière pour éclairer le tissu biologique (10), de sorte que la lumière soit diffusée par le tissu biologique (10) et forme le motif de chatoiement ;
un élément luminescent comprenant un matériau luminescent, configuré pour convertir la lumière diffusée par le tissu biologique (10) en lumière luminescente, par émission de la lumière luminescente lors de son interaction avec la lumière diffusée par le tissu biologique (10) ;
un élément de transformée de Fourier (120, 220, 420) configuré pour transformer la lumière luminescente en un domaine de Fourier spatial au niveau d'un plan de Fourier (121, 221, 421) de l'élément de transformée de Fourier ;
un filtre passe-bande spatial (130, 230, 430) disposé au niveau du plan de Fourier (121, 221, 421), le filtre passe-bande spatial (130, 230, 430) étant configuré pour transmettre sélectivement la lumière à une position spatiale du plan de Fourier (121, 221, 421), la position spatiale correspondant à une bande de tailles de chatoiements du motif de chatoiement ;
un détecteur photosensible (140, 240, 440) configuré pour détecter la lumière transmise à travers le filtre passe-bande (130, 230, 430), dans lequel un signal sorti du détecteur photosensible (140, 240, 440) comprend des informations relatives au contraste du motif de chatoiement.

2. Dispositif (100, 200, 300, 400) selon la revendication 1, dans lequel l'élément de transformée de Fourier (120, 220, 420) comprend une lentille.

3. Dispositif (100, 200, 300, 400) selon la revendication 2, dans lequel la lentille est une lentille sphérique positive plan-convexe.

4. Dispositif (100, 200, 300, 400) selon la revendication 1, dans lequel l'élément de transformée de Fourier (120, 220, 420) comprend un quelconque d'un guide d'ondes, un circuit intégré photonique (PIC), un modulateur spatial de lumière ou un miroir de système microélectromécanique (MEMS).

5. Dispositif (100, 200, 300, 400) selon une quelconque des revendications précédentes, dans lequel le filtre passe-bande spatial (130, 230, 430) est un filtre passe-bande statique configuré pour transmettre sélectivement la lumière à une position spatiale prédéfinie du plan de Fourier (121, 221, 421).

6. Dispositif (100, 200, 300, 400) selon la revendication 5, dans lequel le filtre passe-bande spatial (130, 230, 430) est une ouverture à obstruction annulaire.

7. Dispositif (100, 200, 300, 400) selon une quelconque des revendications 1 à 4, dans lequel le filtre passe-bande spatial (130, 230, 430) est un filtre passe-bande dynamique configuré pour transmettre sélectivement la lumière à une position spatiale variable du plan de Fourier (121, 221, 421).

8. Dispositif (100, 200, 300, 400) selon la revendication 7, dans lequel le filtre passe-bande spatial (130, 230, 430) comprend un d'un écran dynamiquement translucide, un modulateur spatial de lumière ou un miroir de système microélectromécanique (MEMS).

9. Dispositif (100, 200, 300, 400) selon une quelconque des revendications précédentes, dans lequel le filtre passe-bande spatial (130, 230, 430) est un premier filtre passe-bande et dans lequel le dispositif (100, 200, 300, 400) comprend en outre au moins un deuxième filtre passe-bande.

10. Dispositif (200) selon une quelconque des revendications précédentes, dans lequel le dispositif (200) est configuré pour la télédétection et configuré pour éclairer le tissu biologique et collecter la lumière diffusée par le tissu biologique à une distance du tissu biologique comprise entre 0,1 m et 2 m.

11. Dispositif (100, 200, 300, 400) selon une quelconque des revendications précédentes, dans lequel le signal sorti du détecteur photosensible (140, 240, 440) comprend en outre des informations relatives à un nombre de chatoiements du motif de chatoiement.

12. Dispositif (100, 200, 300, 400) selon une quelconque des revendications précédentes, dans lequel le signal sorti du détecteur photosensible (140, 240, 440) comprend en outre des informations relatives aux variations du contraste du motif de chatoiement au fil du temps.

13. Dispositif (100, 200, 300, 400) selon une quelconque des revendications précédentes, dans lequel la source lumineuse (110, 210, 410) est configurée pour générer une lumière au moins partiellement cohérente.

14. Dispositif (100, 200, 300, 400) selon une quelconque des revendications précédentes, dans lequel la source lumineuse (110, 210, 410) est configurée pour générer une lumière d'au moins deux longueurs d'onde.

15. Procédé d'analyse d'un motif de chatoiement provenant d'un tissu biologique, le procédé comprenant :
l'éclairage (S502) du tissu biologique par la lumière d'une source lumineuse, de sorte que la lumière soit diffusée par le tissu biologique et forme le motif de chatoiement ;
la conversion (S503), par un matériau luminescent d'un élément luminescent, de la lumière diffusée par le tissu biologique en lumière luminescente, par émission de lumière luminescente lors de l'interaction avec la lumière diffusée par le tissu biologique ;
la transformation (S506), par un élément de transformée de Fourier, de la lumière luminescente en un domaine spatial de Fourier dans un plan de Fourier de l'élément de transformée de Fourier ;
la transmission sélective (S508), par un filtre passe-bande spatial disposé dans le plan de Fourier, de lumière à une position spatiale du plan de Fourier, la position spatiale correspondant à une bande de tailles de chatoiement du motif de chatoiement ;
la détection (S510), par un détecteur photosensible, de la lumière transmise à travers le filtre passe-bande, dans lequel un signal sorti du détecteur photosensible comprend des informations relatives au contraste sur le motif de chatoiement.
